# EUROPEAN PATENT APPLICATION

(11) **EP 2 952 196 A1**
(43) Date of publication of application: **09.12.2015**
(21) Application number: 14745455.7
(22) Date of filing: 31.01.2014
(51) Int. Cl.: A61K 31/549, A61K 9/20, A61K 31/4184, A61K 47/02, A61K 47/04, A61K 47/18, A61P 7/10, A61P 9/12, A61P 43/00

(54) **MULTILAYER TABLET CONTAINING TELMISARTAN AND HYDROCHLOROTHIAZIDE**

(30) Priority: 31.01.2013 JP 2013016652
(71) Applicant: Sawai Pharmaceutical Co., Ltd., Yodogawa-ku Osaka-shi Osaka 532-0003 (JP)
(72) Inventor: IZUI, Wataru, Osaka City Osaka 532-0003 (JP); OGAWA, Yoshiyuki, Osaka City Osaka 532-0003 (JP); KATSUMOTO, Mami, Osaka City Osaka 532-0003 (JP); HIZAKI, Masaya, Osaka City Osaka 532-0003 (JP)
(74) Representative: Von Kreisler Selting Werner - Partnerschaft von Patentanwälten und Rechtsanwälten mbB
(86) International application number: PCT/JP2014/052361
(87) International publication number: WO 2014/119767

(57) **Abstract**

Provided is a stable multilayer tablet containing telmisartan and hydrochlorothiazide, in which the decomposition of hydrochlorothiazide during storage, etc., is suppressed. A multilayer tablet comprising a first layer containing telmisartan, meglumine, and a moisture-absorbing substance and a second layer containing hydrochlorothiazide is provided. The moisture-absorbing substance may be a porous moisture-absorbing substance. The porous moisture-absorbing substance may be selected from light anhydrous silicic acid, synthetic aluminum silicate, natural aluminum silicate, calcium silicate, magnesium silicate, magnesium aluminosilicate, magnesium aluminometasilicate, hydrated silicon dioxide, and silicon dioxide.

## Description

### TECHNICAL FIELD

The present invention relates to a multilayer tablet composed of two or more layers including a layer containing telmisartan and a layer containing hydrochlorothiazide. The present invention particularly relates to a stable multilayer tablet containing telmisartan and hydrochlorothiazide, in which the decomposition, etc., of hydrochlorothiazide during storage is suppressed.

### BACKGROUND ART

Combination therapy of telmisartan, which is an angiotensin II receptor antagonist, with a diuretic is expected to have a synergistic therapeutic effect in the treatment of hypertension, and thus has been widely applied as a method for treating hypertension. Also in Japan, treatment using telmisartan in combination with hydrochlorothiazide, which is a diuretic, has been provided. As a pharmaceutical preparation used for such treatment, Patent Literature 1, for example, describes a two-layer tablet containing telmisartan and hydrochlorothiazide.

Telmisartan has low solubility under the conditions of physiological pH in the gastrointestinal tract. In order for it to be satisfactorily absorbed by the body, it is necessary to improve the solubility of telmisartan. As a method for improving the solubility of telmisartan, Patent Literatures 1 and 2 describe a method in which telmisartan, basic substances such as meglumine, and a surfactant are dissolved in water or a water/ethanol mixed solution, and granulation is performed using this resultant solution. Meanwhile, it is known that the decomposition of hydrochlorothiazide is promoted under alkali condition. Accordingly, because of the incompatibility of the directly blending of hydrochlorothiazide and a basic substance, which is a component of a telmisartan formulation, a single unit dosage form of telmisartan and hydrochlorothiazide needs to be a two-layer tablet, etc., as described in Patent Literatures 1 and 3.

In addition, it is known that hydrochlorothiazide is easily hydrolyzed under the influence of temperature or humidity and decomposed into 4-amino-6-chloro-1,3-benzenedisulfoneamide. Accordingly, in order to use hydrochlorothiazide as pharmaceuticals, it is necessary to suppress such decomposition of hydrochlorothiazide. Two-layer tablets containing telmisartan and hydrochlorothiazide that are currently available in the market are packaged in a highly moisture-proof substrate for quality preservation. However, in the case of dividing and packaging, etc., such a two-layer tablet has to be stored avoiding high-temperature and high-humidity conditions. Thus, it is hard to say that the stability of such a tablet during storage is sufficiently satisfactory.

### CITATION LIST

### PATENT LITERATURE

Patent Literature 1: Published Japanese Translation of PCT Patent Application No. 2005-514439
Patent Literature 2: Published Japanese Translation of PCT Patent Application No. 2006-502194
Patent Literature 3: Published Japanese Translation of PCT Patent Application No. 2009-517366

### SUMMARY OF INVENTION

### TECHNICAL PROBLEM

The present invention is aimed at solving the problems mentioned above. An object thereof is to provide a stable multilayer tablet containing telmisartan and hydrochlorothiazide, in which the decomposition, etc., of hydrochlorothiazide during storage is suppressed.

### SOLUTION TO PROBLEMS

According to one embodiment of the present invention, a multilayer tablet comprising a first layer containing telmisartan, meglumine, and a moisture-absorbing substance and a second layer containing hydrochlorothiazide is provided.

The moisture-absorbing substance may be a porous moisture-absorbing substance.

The porous moisture-absorbing substance may be selected from light anhydrous silicic acid, synthetic aluminum silicate, natural aluminum silicate, calcium silicate, magnesium silicate, magnesium aluminosilicate, magnesium aluminometasilicate, hydrated silicon dioxide, and silicon dioxide.

### ADVANTAGEOUS EFFECTS OF INVENTION

The present invention provides a stable multilayer tablet containing telmisartan and hydrochlorothiazide, in which the decomposition, etc., of hydrochlorothiazide during storage is suppressed.

### DESCRIPTION OF EMBODIMENTS

Hereinafter, the multilayer tablet containing telmisartan and hydrochlorothiazide according to the present invention will be described. However, the multilayer tablet containing telmisartan and hydrochlorothiazide of the present invention is not limited to the description in the following embodiments and examples.

The present inventors have conducted extensive research on the cause of the decomposition of hydrochlorothiazide into 4-amino-6-chloro-1,3-benzenedisulfoneamide (hereinafter referred to as DSA) in a two-layer tablet containing telmisartan and hydrochlorothiazide (hereinafter referred to as TLS/HCTZ two-layer tablet). As a part of the research, they tested the stability of TLS/HCTZ two-layer tablets (temperature: 40°C, humidity: 75%RH, stored for two weeks). As a result, 10% or more of DSA was detected from a conventional TLS/HCTZ two-layer tablet, confirming the decomposition of hydrochlorothiazide with respect to the conventional TLS/HCTZ two-layer tablet. Meanwhile, a tablet, which consists of only the components of the layer containing hydrochlorothiazide of the conventional TLS/HCTZ two-layer tablet mentioned above, was produced (a single active ingredient preparation of hydrochlorothiazide) and the stability thereof was tested under the same conditions. As a result, only less than 1 % of DSA was detected. From these results, the present inventors thought that the factor that causes the decomposition of hydrochlorothiazide in a TLS/HCTZ two-layer tablet would be in the layer containing telmisartan.

As mentioned above, it is known that the hydrolysis of hydrochlorothiazide is promoted by temperature and humidity. Meanwhile, as shown by the above test results, decomposition of hydrochlorothiazide was hardly observed in the single active ingredient preparation of hydrochlorothiazide. Accordingly, it was likely that in a TLS/HCTZ two-layer tablet, moisture included in a layer other than the layer containing hydrochlorothiazide affects hydrochlorothiazide. It was surmised that moisture included in a layer other than the layer containing hydrochlorothiazide in a TLS/HCTZ two-layer tablet, that is, moisture absorbed by the layer containing telmisartan, transfers from the layer containing telmisartan to the layer containing hydrochlorothiazide. As a result of further research, the present inventors have found out that although the moisture absorbency of telmisartan itself is relatively low, a salt form of telmisartan and meglumine (telmisartan meglumine salt), which is a basic substance, has about 10 times the moisture absorbency of telmisartan itself.

As mentioned above, meglumine is one of the components necessary to improve the solubility of telmisartan. In the related art, in order to improve the solubility of telmisartan, telmisartan and meglumine, etc., are dissolved in water or a water/ethanol mixed solution, and granulation is performed using this resultant solution; a telmisartan meglumine salt is produced during this granulation process. The present inventors have found that the resulting telmisartan meglumine salt has high moisture absorbency, and further found that, surprisingly, the high moisture absorbency of the telmisartan meglumine salt is the factor that causes the decomposition of hydrochlorothiazide in a TLS/HCTZ two-layer tablet. This finding has not been reported in the past and is reported by the present inventors for the first time.

The present inventors have found the new problem that an increase in the moisture absorption of a layer containing telmisartan causes the decomposition of hydrochlorothiazide present in another layer. Further, to deal with this new problem, the present inventors have found that the decomposition of hydrochlorothiazide can be suppressed when a substance that adsorbs moisture is added to the layer containing telmisartan, and thus accomplished the present invention. The present invention reports, for the first time, that in a multilayer tablet composed of two or more layers including a layer containing telmisartan and a layer containing hydrochlorothiazide (hereinafter referred to as TLS/HCTZ multilayer tablet), when a moisture-absorbing substance is added to the layer containing telmisartan, hydrochlorothiazide is stabilized.

The TLS/HCTZ multilayer tablet according to the present invention is composed of two or more layers including a first layer containing telmisartan, meglumine, and a moisture-absorbing substance and a second layer containing hydrochlorothiazide. The multilayer tablet according to the present invention should just be composed of a plurality of layers, and may be a laminated tablet having layers that are vertically laminated, a dry-coated tablet having an inner layer (inner core) and an outer layer, etc. Examples of laminated tablets include two-layer tablets.

In this embodiment, the TLS/HCTZ multilayer tablet contains a predetermined amount of telmisartan in the first layer, and the content of telmisartan is 40 mg or 80 mg/tablet, for example. In addition, in this embodiment, the TLS/HCTZ multilayer tablet contains a predetermined amount of meglumine in the first layer. The content of meglumine should just be an amount necessary to improve the solubility of telmisartan and may be, for example, the same as the content of telmisartan, that is, 40 mg or 80 mg/tablet. In addition, in the formulated TLS/HCTZ multilayer tablet, in order for the entire telmisartan to be present as a telmisartan meglumine salt, the content of meglumine should just be equimolar to or greater than the amount of telmisartan. Incidentally, in the TLS/HCTZ multilayer tablet according to the present invention, the amounts of telmisartan and meglumine contained in the first layer are not limited thereto and may be arbitrarily changed.

In this embodiment, the TLS/HCTZ multilayer tablet contains a predetermined amount of moisture-absorbing substance in the first layer. It is preferable that the moisture-absorbing substance is a porous moisture-absorbing substance, and the porous moisture-absorbing substance may be selected, for example, from light anhydrous silicic acid, synthetic aluminum silicate, natural aluminum silicate, calcium silicate, magnesium silicate, magnesium aluminosilicate, magnesium aluminometasilicate, hydrated silicon dioxide, silicon dioxide, etc. In addition to these porous moisture-absorbing substances, the moisture-absorbing substance according to the present invention may also be selected from compound aluminum potassium silicate granules, crystalline cellulose, aluminum oxide, aluminum hydroxide, magnesium carbonate, precipitated calcium carbonate, dextrin, bentonite, lauryl methacrylate, medicinal carbon, diatomaceous earth, kaolin, carmellose calcium, acid anhydrides, etc., but is not limited thereto. In this embodiment, it is particularly preferable to use a porous moisture-absorbing substance. This is because a porous moisture-absorbing substance physically adsorbs moisture, also is chemically stable even after adsorbing moisture, and further has a large surface area and thus exerts high water absorbability even when contained in a small amount.

In this embodiment, the content of moisture-absorbing substance in the first layer should just be an amount that can significantly suppress the decomposition of hydrochlorothiazide, and may be arbitrarily set according to the kind of moisture-absorbing substance. The content of moisture-absorbing substance in the first layer is, for example, based on 100 wt% of the entire first layer, 0.1 wt% or more and 25 wt% or less, preferably 0.5 wt% or more and 10 wt% or less, and more preferably 1 wt% or more and 5 wt% or less.

In this embodiment, the first layer of the TLS/HCTZ multilayer tablet also contains a water-soluble diluent, an excipient, and/or an adjuvant. Examples of usable water-soluble diluents include known carbohydrates, including monosaccharides such as glucose, oligosaccharides such as sucrose, anhydrous lactose, and lactose monohydrate, sugar alcohols such as sorbitol, erythritol, mannitol, dulcitol, ribitol, and xylitol, and like. In this embodiment, it is preferable to use mannitol having low moisture absorbency as a water-soluble diluent.

Examples of other excipients and/or adjuvants include binders, disintegrants, diluents, carriers, fillers, lubricants, fluidizers, colorants, pH adjusting agents, surfactants, and emulsifying agents. In this embodiment, as binders, examples of dry binders include crystalline cellulose. In addition, examples of wet granulation binders include corn starch, polyvinylpyrrolidone (povidone), polyvinylpyrrolidone-vinyl acetate copolymer (copovidone), macrogol, and cellulose derivatives such as hydroxymethylcellulose, hydroxyethylcellulose, hydroxypropylcellulose, and hydroxypropylmethylcellulose.

In this embodiment, examples of disintegrants include sodium carboxymethyl starch, crospovidone, croscarmellose sodium, carboxymethylcellulose sodium, dry corn starch, low-substituted hydroxypropylcellulose, carmellose calcium, carmellose sodium, carmellose, and crystalline cellulose.

Examples of diluents and carriers include powdered cellulose, crystalline cellulose, cellulose derivatives such as hydroxymethylcellulose, hydroxyethylcellulose, hydroxypropylcellulose, and hydroxypropylmethylcellulose, dibasic calcium phosphate, corn starch, pregelatinized starch, and polyvinylpyrrolidone (povidone), etc. Examples of lubricants include stearic acid, magnesium stearate, sodium stearyl fumarate, and glycerol tribehenate, etc. Examples of fluidizers include colloidal silica and talc, etc. Examples of colorants including dyes and pigments include iron oxide red or yellow, titanium dioxide, and talc, etc. Examples of pH adjusting agents include citric acid, tartaric acid, fumaric acid, sodium citrate, dibasic calcium phosphate, and dibasic sodium phosphate, etc. Examples of surfactants and emulsifying agents include poloxamer (Pluronic), lauromacrogol, polysorbate, sodium lauryl sulfate, polyethoxylated castor oil, and hydrogenated castor oil, etc. It is also possible to select a mixture of two or more kinds of these excipients and/or adjuvants.

In this embodiment, the TLS/HCTZ multilayer tablet contains a predetermined amount of hydrochlorothiazide in the second layer, and the content of hydrochlorothiazide is 12.5 mg, for example. Incidentally, in the TLS/HCTZ multilayer tablet according to the present invention, the amount of hydrochlorothiazide contained in the second layer is not limited thereto and may be arbitrarily changed.

In this embodiment, the second layer of the TLS/HCTZ multilayer tablet contains a filler, a binder, a disintegrant, and a lubricant. In addition, the second layer of the TLS/HCTZ multilayer tablet may also arbitrarily contain other excipients and adjuvants. Fillers may be known fillers, and may be selected, for example, from carbohydrates including crystalline cellulose, saccharides such as glucose, lactose, and sucrose, sugar alcohols such as sorbitol, erythritol, mannitol, dulcitol, ribitol, and xylitol, and like. Binders may be selected, for example, from powdered cellulose, crystalline cellulose, corn starch, polyvinylpyrrolidone (povidone), polyvinyl pyrrolidone-vinyl acetate copolymer (copovidone), cellulose derivatives such as hydroxymethylcellulose, hydroxyethylcellulose, hydroxypropylcellulose, and hydroxypropylmethylcellulose, etc. Disintegrants may be selected, for example, from sodium carboxymethyl starch, crospovidone, croscarmellose sodium, carboxymethylcellulose sodium, dry corn starch, etc. Lubricants may be selected, for example, from stearic acid, magnesium stearate, sodium stearyl fumarate, glycerol tribehenate, etc.

Examples of excipients and/or adjuvants include diluents, carriers, fluidizers, colorants, pH adjusting agents, surfactants, and emulsifying agents. Diluents and carriers may be selected, for example, from powdered cellulose, crystalline cellulose, cellulose derivatives such as hydroxymethylcellulose, hydroxyethylcellulose, hydroxypropylcellulose, and hydroxypropylmethylcellulose, dibasic calcium phosphate, corn starch, pregelatinized starch, polyvinylpyrrolidone (povidone), etc. Fluidizers may be selected, for example, from colloidal silica, talc, etc. Colorants including dyes and pigments may be selected, for example, from iron oxide red or yellow, titanium dioxide, talc, etc. pH adjusting agents may be selected, for example, from citric acid, tartaric acid, fumaric acid, sodium citrate, dibasic calcium phosphate, dibasic sodium phosphate, etc. Surfactants and emulsifying agents may be selected, for example, from poloxamer (Pluronic), polyethylene glycol, carboxymethylcellulose sodium, polyethoxylated and hydrogenated castor oils, etc. It is also possible to select a mixture of two or more kinds of these excipients and/or adjuvants.

In the TLS/HCTZ multilayer tablet according to the present invention, because a predetermined amount of moisture-absorbing substance is added to the first layer containing telmisartan and meglumine, external moisture is adsorbed by the moisture-absorbing substance, whereby the decomposition of hydrochlorothiazide due to moisture can be suppressed. This makes it possible to provide a stable multilayer tablet containing telmisartan and hydrochlorothiazide, in which the decomposition, etc., of hydrochlorothiazide is suppressed even during storage.

### <Production Method>

The TLS/HCTZ multilayer tablet according to the present invention can be produced in accordance with a multilayer tablet production method known in the field of pharmaceutical sciences. For example, a water-soluble diluent is granulated using an aqueous solution containing telmisartan, meglumine, and a surfactant to give a granulated product. The particle size of the obtained granulated product is regulated to give a particle-size-regulated product, and then a moisture-absorbing substance and a lubricant are mixed therewith to give a ready-for-tableting mixture for the first layer that is ready for tableting.

Meanwhile, hydrochlorothiazide and a water-soluble diluent are mixed, and the resultant mixture is granulated using an aqueous solution containing a binder to give a granulated product. The particle size of the obtained granulated product is regulated to give a particle-size-regulated product, and then a lubricant is mixed therewith to give a ready-for-tableting mixture for the second layer that is ready for tableting. The ready-for-tableting mixture for the first layer is placed in a tablet press, and then the ready-for-tableting mixture for the second layer is placed therein, followed by tableting, whereby the TLS/HCTZ multilayer tablet according to the present invention can be produced. Incidentally, tableting may be performed using a commercially available tablet press.

### EXAMPLES

The TLS/HCTZ multilayer tablet according to the present invention mentioned above will be described in further detail through specific production examples and test results.

### <Example 1 >

D-mannitol (304.0 g) was mixed by a fluidized-bed granulator (manufactured by Powrex Corp., Model: MP-01) at an air supply temperature of 90°C. An aqueous solution (728.0 g) of telmisartan (160.0 g), meglumine (160.0 g), macrogol 6000 (32.0 g), and lauromacrogol (16.0 g) was sprayed thereto at about 3.3 g/min, thereby giving a granulated product. The particle size of the granulated product was then regulated through a No. 22 sieve, thereby giving a particle-size-regulated product (1) for the first layer. Light anhydrous silicic acid (Adsolider® 101 (Freund Corporation), 3.4 g) and magnesium stearate (5.6 g) were mixed with the particle-size-regulated product (1), thereby giving a ready-for-tableting mixture (1) for the first layer.

Hydrochlorothiazide (37.5 g), D-mannitol (304.5 g), and crystalline cellulose (66.0 g) were mixed by a fluidized-bed granulator (manufactured by Powrex Corp., Model: MP-01) at an air supply temperature of 90°C. An aqueous solution (180.0 g) of hydroxypropylcellulose (9.0 g) was sprayed thereto at about 4.5 g/min, thereby giving a granulated product. The particle size of granulated product was regulated through a No. 22 sieve, thereby giving a particle-size-regulated product (2) for the second layer. Magnesium stearate (3.0 g) was mixed with the particle-size-regulated product (2), thereby giving a ready-for-tableting mixture (2) for the second layer. The ready-for-tableting mixture (1) for the first layer was placed in a tablet press, and then the ready-for-tableting mixture (2) for the second layer was placed therein, followed by tableting, thereby giving two-layer tablets each weighing 480.5 mg.

### <Example 2>

Light anhydrous silicic acid (Adsolider 101, 6.8 g) and magnesium stearate (5.60 g) were mixed with the particle-size-regulated product (1) (672.0 g) for the first layer obtained in Example 1, thereby giving a ready-for-tableting mixture (3). The ready-for-tableting mixture (3) was placed in a tablet press, and then the ready-for-tableting mixture (2) obtained in Example 1 was placed therein, followed by tableting, thereby giving two-layer tablets each weighing 482.2 mg.

### <Example 3>

Light anhydrous silicic acid (Adsolider 101, 21.0 g) and magnesium stearate (5.60 g) were mixed with the particle-size-regulated product (1) (672.0 g) obtained in Example 1, thereby giving a ready-for-tableting mixture (4). The ready-for-tableting mixture (4) was placed in a tablet press, and then the ready-for-tableting mixture (2) obtained in Example 1 was placed therein, followed by tableting, thereby giving two-layer tablets each weighing 489.3 mg.

### <Example 4>

Light anhydrous silicic acid (Adsolider 101, 35.6 g) and magnesium stearate (5.60 g) were mixed with the particle-size-regulated product (1) (672.0 g) obtained in Example 1, thereby giving a ready-for-tableting mixture (5). The ready-for-tableting mixture (5) was placed in a tablet press, and then the ready-for-tableting mixture (2) obtained in Example 1 was placed therein, followed by tableting, thereby giving two-layer tablets each weighing 496.6 mg.

### <Example 5>

Light anhydrous silicic acid (AEROSIL® 200 (Nippon Aerosil), 21.0 g) and magnesium stearate (5.60 g) were mixed with the particle-size-regulated product (1) (672.0 g) obtained in Example 1, thereby giving a ready-for-tableting mixture (4-1). The ready-for-tableting mixture (4-1) was placed in a tablet press, and then the ready-for-tableting mixture (2) obtained in Example 1 was placed therein, followed by tableting, thereby giving two-layer tablets each weighing 489.3 mg.

### <Example 6>

Magnesium aluminometasilicate (Neusilin® UFL2 (Fuji Chemical), 21.0 g) and magnesium stearate (5.60 g) were mixed with the particle-size-regulated product (1) (672.0 g) obtained in Example 1, thereby giving a ready-for-tableting mixture (4-2). The ready-for-tableting mixture (4-2) was placed in a tablet press, and then the ready-for-tableting mixture (2) obtained in Example 1 was placed therein, followed by tableting, thereby giving two-layer tablets each weighing 489.3 mg.

### <Example 7>

Calcium silicate (FLORITE® (Tomita Pharmaceutical), 21.0 g) and magnesium stearate (5.60 g) were mixed with the particle-size-regulated product (1) (672.0 g) obtained in Example 1, thereby giving a ready-for-tableting mixture (4-3). The ready-for-tableting mixture (4-3) was placed in a tablet press, and then the ready-for-tableting mixture (2) obtained in Example 1 was placed therein, followed by tableting, thereby giving two-layer tablets each weighing 489.3 mg.

### <Example 8>

Hydrated silicon dioxide (Carplex® (DSL Japan), 21.0 g) and magnesium stearate (5.60 g) were mixed with the particle-size-regulated product (1) (672.0 g) obtained in Example 1, thereby giving a ready-for-tableting mixture (4-4). The ready-for-tableting mixture (4-4) was placed in a tablet press, and then the ready-for-tableting mixture (2) obtained in Example 1 was placed therein, followed by tableting, thereby giving two-layer tablets each weighing 489.3 mg.

### <Example 9>

Synthetic aluminum silicate (Kyowa Chemical Industry Co., Ltd., 21.0 g) and magnesium stearate (5.60 g) were mixed with the particle-size-regulated product (1) (672.0 g) obtained in Example 1, thereby giving a ready-for-tableting mixture (4-5). The ready-for-tableting mixture (4-5) was placed in a tablet press, and then the ready-for-tableting mixture (2) obtained in Example 1 was placed therein, followed by tableting, thereby giving two-layer tablets each weighing 489.3 mg.

### <Example 10>

Magnesium silicate (Kyowa Chemical Industry Co., Ltd., 21.0 g) and magnesium stearate (5.60 g) were mixed with the particle-size-regulated product (1) (672.0 g) obtained in Example 1, thereby giving a ready-for-tableting mixture (4-6). The ready-for-tableting mixture (4-6) was placed in a tablet press, and then the ready-for-tableting mixture (2) obtained in Example 1 was placed therein, followed by tableting, thereby giving two-layer tablets each weighing 489.3 mg.

### <Comparative Example 1>

Magnesium stearate (5.60 g) was mixed with the particle-size-regulated product (1) (672.0 g) obtained in Example 1, thereby giving a ready-for-tableting mixture (6). The ready-for-tableting mixture (6) was placed in a tablet press, and then the ready-for-tableting mixture (2) obtained in Example 1 was placed therein, followed by tableting, thereby giving two-layer tablets each weighing 478.8 mg.

### <Comparative Example 2>

Light anhydrous silicic acid (Adsolider 101, 2.1 g) and magnesium stearate (3.0 g) were mixed with the particle-size-regulated product (2) (417.0 g) obtained in Example 1, thereby giving a ready-for-tableting mixture (7). The ready-for-tableting mixture (6) obtained in Comparative Example 1 was placed in a tablet press, and then the ready-for-tableting mixture (7) was placed therein, followed by tableting, thereby giving two-layer tablets each weighing 479.5 mg.

### <Comparative Example 3>

Light anhydrous silicic acid (Adsolider 101, 4.2 g) and magnesium stearate (3.0 g) were mixed with the particle-size-regulated product (2) (417.0 g) obtained in Example 1, thereby giving a ready-for-tableting mixture (8). The ready-for-tableting mixture (6) obtained in Comparative Example 1 was placed in a tablet press, and then the ready-for-tableting mixture (8) was placed therein, followed by tableting, thereby giving two-layer tablets each weighing 480.2 mg.

### <Comparative Example 4>

Light anhydrous silicic acid (Adsolider 101, 12.9 g) and magnesium stearate (3.0 g) were mixed with the particle-size-regulated product (2) (417.0 g) obtained in Example 1, thereby giving a ready-for-tableting mixture (9). The ready-for-tableting mixture (6) obtained in Comparative Example 1 was placed in a tablet press, and then the ready-for-tableting mixture (9) was placed therein, followed by tableting, thereby giving two-layer tablets each weighing 483.1 mg.

### <Comparative Example 5>

Light anhydrous silicic acid (Adsolider 101, 22.2 g) and magnesium stearate (3.0 g) were mixed with the particle-size-regulated product (2) (417.0 g) obtained in Example 1, thereby giving a ready-for-tableting mixture (10). The ready-for-tableting mixture (6) obtained in Comparative Example 1 was placed in a tablet press, and then the ready-for-tableting mixture (10) was placed therein, followed by tableting, thereby giving two-layer tablets each weighing 486.2 mg.

### <Stability Test 1>

The two-layer tablets obtained in the above Examples 1 to 4 and Comparative Examples 1 to 5 were evaluated for stability. For the evaluation of stability, each two-layer tablet was stored at 40°C and 75%RH for two weeks, and then a purity test was performed by using liquid chromatography. The ratio of a related substance was calculated as follows. With respect to hydrochlorothiazide-derived related substance, the ratio was calculated from the peak area thereof relative to the peak area of hydrochlorothiazide. With respect to telmisartan-derived related substance, the ratio was calculated from the peak area thereof relative to the peak area of telmisartan. The total of the ratios of both hydrochlorothiazide-derived and telmisartan-derived related substances was defined as Total Related Substance. The measurement results of the examples of the present invention are shown in Table 1, while the measurement results of the comparative examples are shown in Table 2.

**[Table 1]**

| | | Comparative Example 1 | Example 1 | Example 2 | Example 3 | Example 4 |
|---|---|---|---|---|---|---|
| Adsolider (%) | First layer | 0 | 0.5 | 1 | 3 | 5 |
| | Second layer | 0 | 0 | 0 | 0 | 0 |
| Total Substance | Related (%) | 7.76 | 7.43 | 7.27 | 5.84 | 5.62 |
| DSA (%) | | 7.61 | 7.25 | 7.12 | 5.72 | 5.47 |

The amount of Adsolider in the table means the amount of Adsolider added to each layer.

**[Table 2]**

| Comparative Example | | 1 | 2 | 3 | 4 | 5 |
|---|---|---|---|---|---|---|
| Adsolider (%) | First layer | 0 | 0 | 0 | 0 | 0 |
| | Second layer | 0 | 0.5 | 1 | 3 | 5 |
| Total Related Substance (%) | | 7.76 | 7.41 | 8.73 | 12.23 | 12.26 |
| DSA (%) | | 7.61 | 7.28 | 8.56 | 11.93 | 14.93 |

The amount of Adsolider in the table means the amount of Adsolider added to each layer.

### <Stability Test 2>

The two-layer tablets obtained in the above Examples 3 and 5 to 10 were evaluated for stability. The evaluation of stability was performed in the same manner as in Stability Test 1. The measurement results are shown in Table 3.

**[Table 3]**

| Example | | 3 | 5 | 6 | 7 | 8 | 9 | 10 |
|---|---|---|---|---|---|---|---|---|
| Porous Substance | Adsolider 101 | 3 | 0 | 0 | 0 | 0 | 0 | 0 |
| (%) | AEROSIL 200 | 0 | 3 | 0 | 0 | 0 | 0 | 0 |
| | Neusilin UFL2 | 0 | 0 | 3 | 0 | 0 | 0 | 0 |
| | FLORITE | 0 | 0 | 0 | 3 | 0 | 0 | 0 |
| | Carplex | 0 | 0 | 0 | 0 | 3 | 0 | 0 |
| | Synthetic Aluminum Silicate | 0 | 0 | 0 | 0 | 0 | 3 | 0 |
| | Magnesium Silicate | 0 | 0 | 0 | 0 | 0 | 0 | 3 |
| Total Related Substance (%) | | 5.84 | 6.08 | 5.32 | 6.30 | 5.83 | 5.72 | 6.67 |
| DSA (%) | | 5.72 | 5.82 | 5.18 | 6.14 | 5.68 | 5.58 | 6.53 |

The amount of porous substance in the table means the amount of porous moisture-absorbing substance added to the first layer.

As is clear from Table 1, as compared with Comparative Example 1 where no moisture-absorbing substance was added, in Examples 1 to 4 where light anhydrous silicic acid (Adsolider 101), which is a moisture-absorbing substance, was added to the first layer containing telmisartan, the production of DSA, which is a decomposition product of hydrochlorothiazide, was suppressed. Further, in Examples 1 to 4, the production of DSA decreased with an increase in the amount of light anhydrous silicic acid (Adsolider 101), which is a moisture-absorbing substance. That is, the decomposition of hydrochlorothiazide contained in the second layer was suppressed with an increase in the amount of moisture-absorbing substance added to the first layer together with telmisartan and meglumine. Meanwhile, as is clear from Table 2, in Comparative Examples 2 to 5 where light anhydrous silicic acid (Adsolider 101), which is a moisture-absorbing substance, was added to the second layer, the decomposition of hydrochlorothiazide was not suppressed with an increase in the amount of moisture-absorbing substance added to the second layer together with hydrochlorothiazide, but the decomposition rather tended to be promoted.

In addition, as is clear from Table 3, also in Example 5 where the kind of light anhydrous silicic acid added as a moisture-absorbing substance was changed to AEROSIL 200, Example 6 where magnesium aluminometasilicate was added as a moisture-absorbing substance, Example 7 where calcium silicate was added as a moisture-absorbing substance, Example 8 where hydrated silicon dioxide was added as a moisture-absorbing substance, Example 9 where synthetic aluminum silicate was added as a moisture-absorbing substance, and Example 10 where magnesium silicate was added as a moisture-absorbing substance, the decomposition of hydrochlorothiazide contained in the second layer was suppressed by the addition of a moisture-absorbing substance to the first layer together with telmisartan and meglumine.

As described above, in the TLS/HCTZ multilayer tablet according to this examples, when a predetermined amount of moisture-absorbing substance is added to the first layer containing telmisartan and meglumine, the decomposition of hydrochlorothiazide contained in the second layer can be suppressed. The same effects can also be obtained even when the kind of moisture-absorbing substance added is changed.

## Claims

1. A multilayer tablet **characterized by** comprising:
a first layer containing telmisartan, meglumine, and a moisture-absorbing substance; and
a second layer containing hydrochlorothiazide.

2. The multilayer tablet according to claim 1, **characterized in that** the moisture-absorbing substance is a porous moisture-absorbing substance.

3. The multilayer tablet according to claim 2, **characterized in that** the porous moisture-absorbing substance is selected from light anhydrous silicic acid, synthetic aluminum silicate, natural aluminum silicate, calcium silicate, magnesium silicate, magnesium aluminosilicate, magnesium aluminometasilicate, hydrated silicon dioxide, and silicon dioxide.
